(19) Europäisches Patentamt
European Patent Office
Office européen des brevets

(11) **EP 3 632 328 B1**

(12) **EUROPEAN PATENT SPECIFICATION**

(45) Date of publication and mention
of the grant of the patent:
**06.04.2022 Bulletin 2022/14**

(21) Application number: **19762285.5**

(22) Date of filing: **16.05.2019**

(51) International Patent Classification (IPC):
$A61B\ 6/03$ (2006.01)   $G01T\ 1/24$ (2006.01)
$A61B\ 6/00$ (2006.01)   $G06N\ 3/00$ (2006.01)
$G06N\ 20/00$ (2019.01)   $H04N\ 5/32$ (2006.01)
$H05G\ 1/00$ (2006.01)   $H05H\ 9/00$ (2006.01)
$G06N\ 3/08$ (2006.01)

(52) Cooperative Patent Classification (CPC):
**A61B 6/4241; A61B 6/482; A61B 6/488;
A61B 6/545; G06N 3/084; H04N 5/32**

(86) International application number:
**PCT/CN2019/087220**

(87) International publication number:
**WO 2020/034679 (20.02.2020 Gazette 2020/08)**

(54) **THRESHOLD VALUE OPTIMIZING METHOD AND APPARATUS BASED ON K-EDGE IMAGING, DEVICE, AND MEDIUM**

VERFAHREN, EINRICHTUNG, VORRICHTUNG UND MEDIUM ZUR OPTIMIERUNG VON SCHWELLENWERTEN AUF BASIS VON K-KANTEN-BILDGEBUNG

PROCÉDÉ D'OPTIMISATION DE VALEUR SEUIL ET APPAREIL BASÉ SUR L'IMAGERIE DE BORD K, DISPOSITIF, ET MILIEU

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **17.08.2018 CN 201810942770**

(43) Date of publication of application:
**08.04.2020 Bulletin 2020/15**

(73) Proprietors:
• **Nuctech Company Limited
TongFang Building,
Shuangqinglu,
Haidian District
Beijing 100084 (CN)**
• **Tsinghua University
Beijing 100084 (CN)**

(72) Inventors:
• **XING, Yuxiang
Shuangqinglu, Haidan District
Beijing 100084 (CN)**

• **HUANG, Kaixin
Shuangqinglu, Haidan District
Beijing 100084 (CN)**
• **ZHANG, Li
Shuangqinglu, Haidan District
Beijing 100084 (CN)**
• **SHEN, Le
Shuangqinglu, Haidan District
Beijing 100084 (CN)**
• **DENG, Zhi
Shuangqinglu, Haidan District
Beijing 100084 (CN)**
• **CHEN, Zhiqiang
Shuangqinglu, Haidan District
Beijing 100084 (CN)**
• **LIU, Yinong
Shuangqinglu, Haidan District
Beijing 100084 (CN)**

(74) Representative: **Boult Wade Tennant LLP
Salisbury Square House
8 Salisbury Square
London EC4Y 8AP (GB)**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(56) References cited:
WO-A1-2005/092187    CN-A- 101 410 871
CN-A- 102 076 263    CN-A- 102 088 907
CN-A- 102 834 736    CN-A- 102 920 434
CN-A- 103 654 832    US-A1- 2014 023 181
US-A1- 2016 054 453    US-A1- 2016 206 256
US-A1- 2017 086 775    US-B1- 6 408 050

## Description

### TECHNICAL FIELD

The present application relates to the field of energy spectrum Computed Tomography (CT), and more particularly, to a method, apparatus, device and medium for optimizing thresholds based on K-edge imaging.

### BACKGROUND

For energy spectrum K-edge imaging, a photon counting detector is required. However, once a photon counting detector is used, it is necessary to determine thresholds of the photon counting detector so as to obtain images with higher quality.

Currently, there are two empirical methods for threshold selection: one is to divide the energy uniformly across the entire energy spectrum range, that is, to make a width of each energy window roughly the same. The other is to divide the number of photons uniformly, that is, to make the number of photons in each energy window roughly the same, but this method requires the energy spectrum to be known.

Both the above methods are not able to select the optimal thresholds for the photon counting detector if a composition of the object to be detected is unknown.

Document US2016054453 A1 discloses a method and device comprising an identifying unit which identifies, as energy bins to be used for material decomposition among the energy bins, and which may identify, as energy bins to be used for the material decomposition among the energy bins, energy bins that do not overlap the K absorption edge of a decomposition target material.

### SUMMARY

Embodiments of the present disclosure may provide a method, apparatus, device and medium for optimizing thresholds based on K-edge imaging, which may be able to select the optimal thresholds for a photon counting detector even a composition of the object to be detected is unknown.

According to a first aspect of embodiments of the present disclosure, a method for optimizing thresholds based on K-edge imaging may be provided, comprising:

pre-scanning, by using a photon counting detector, an object to be detected according to a preset threshold group, to obtain first detection data detection data;

determining, according to the first detection data, line integral values of decomposition coefficients of individual basis functions in a basis function combination of each ray, wherein each ray has a fixed basis function combination;

selecting, according to the line integral values of the decomposition coefficients of the individual basis functions in the basis function combination of the each ray, rays of which a line integral value of a contrast agent material basis function in the fixed basis function combination is greater than a preset line integration threshold;

inputting the detection data corresponding to each of the selected rays to a pre-trained neural network model, to obtain a local optimal threshold group of each of the selected rays; and

determining, based on local optimal threshold groups, a global optimal threshold group for the K-edge imaging of the photon counting detector.

According to a second aspect of embodiments of the present disclosure, an apparatus for optimizing thresholds based on K-edge imaging may be provided, comprising:

a line integral value determining module, a selecting module, a pre-trained neural network model module and an integrating module;

the line integral value determining module is configured to determine, according to first detection data, line integral values of decomposition coefficients of individual basis functions in a basis function combination of each ray, wherein each ray has a fixed basis function combination; and wherein, the first detection data is obtained by using a photon counting detector to pre-scan an object to be detected according to a preset threshold group;

the selecting module is configured to select, according to the line integral values of the decomposition coefficients of the individual basis functions in the basis function combination of the each ray, rays of which a line integral value of a contrast agent material basis function in the fixed basis function combination is greater than a preset line integration threshold;

the pre-trained neural network model module is configured to input the detection data corresponding to each of the selected rays to a pre-trained neural network model, to obtain a local optimal threshold group of each of the selected rays; and

the integrating module is configured to determine, based on local optimal threshold groups, a global optimal threshold group for the K-edge imaging of the photon counting detector.

According to a third aspect of the embodiments of the present disclosure, a terminal device may be provided, comprising:

a memory, a processor, a communication interface, and a bus, wherein:

the memory, the processor, the communication interface connects and communicates with each other through the bus;

the memory is configured to store executable program codes;

the processor is configured to run a program corresponding to the executable program codes stored in the memory by reading the executable program codes, to perform the method for optimizing thresholds according to the above first aspect.

According to a fourth aspect of embodiments of the present disclosure, a computer storage medium comprising instructions may be provided, when executed on a computer, the instructions cause the computer to perform the method for optimizing thresholds according to the above first aspect.

The method, apparatus, device and medium for optimizing thresholds based on K-edge imaging according to embodiments of the present disclosure may roughly estimate an internal structure of the object to be detected using a low dose pre-scan form, thereby reducing the damage to the object to be detected. Secondly, the global optimal threshold group may be obtained effectively for an unknown object to be detected. Further, the use of the pre-trained neural network module to obtain the local optimal threshold group may improve the efficiency significantly.

## BRIEF DESCRIPTION OF THE DRAWINGS

The present disclosure may be better understood from the following description of specific embodiments of the present disclosure in conjunction with the accompanying drawings, wherein:

other features, objects and advantages of the present disclosure will become apparent by reading the following detailed description of non-limiting embodiments referring to the accompanying drawings, wherein the same or similar reference numerals represent the same or similar features.

Fig. 1 is a flow chart showing a method for optimizing thresholds based on K-edge imaging according to an embodiment of the present disclosure;

Fig. 2 is a schematic diagram showing a preset threshold group in an embodiment of the present disclosure;

Fig. 3 is a detailed flowchart showing a method for optimizing thresholds according to another embodiment of the present disclosure;

Fig. 4 is a schematic diagram showing a statistical local optimal threshold group in an embodiment of the present disclosure;

Fig. 5 is a structural schematic diagram showing a training motif used in an embodiment of the present disclosure;

Fig. 6 is a structural schematic diagram showing a pre-trained neural network model in an embodiment of the present disclosure;

Fig. 7 is a structural schematic diagram showing a testing motif used in an embodiment of the present disclosure;

Fig. 8 is a structural schematic diagram showing an apparatus for optimizing thresholds based on K-edge imaging provided by an embodiment of the present disclosure;

Fig. 9 is a structural schematic diagram showing an apparatus for optimizing thresholds according to another embodiment of the present disclosure;

Fig. 10 is a block diagram showing an exemplary hardware architecture of a computing device capable of implementing a method and apparatus for optimizing thresholds in accordance with embodiments of the present disclosure.

## DETAILED DESCRIPTION

The features of various aspects and exemplary embodiments of the present disclosure will be described below in detail. In the following detailed description, numerous specific details are set forth in order to provide a thorough understanding of the present disclosure. However, it will be apparent to those skilled in the art that the present disclosure may be practiced without some of these specific details. The following description of the embodiments is merely intended to provide a better understanding of the present disclosure by illustrating examples of the present disclosure. In the accompanying drawings and the following description, at least part of well-known structures and techniques are not illustrated as so to avoid unnecessarily obscuring the present disclosure; moreover, the dimension of some of the structures may be exaggerated for clarity. The same reference numerals in the drawings represent the same or similar structures, and thus their detailed description will be omitted. Furthermore, the features, structures, or characteristics described hereinafter may be incorporated in one or more embodiments in any suitable manner.

With the development of detector technology and electronics technology, a new type of detector called Photon Counting Detector (PCD) has emerged. By setting different thresholds, the PCD may count the photons whose energy is higher than the set thresholds, so as to count each incident photon by intervals, causing the original CT data carries the information of the energy dimension. PCD may not only reduce noise by setting appropriate thresholds, but also collect data of multiple energy windows at the same time, which is more convenient for data processing. The CT using detection data under multiple sets of thresholds may be collectively referred to as energy spectrum CT. Among a variety of applications of the energy spectrum CT, K-edge imaging is a very important one.

The energy of the K-edge is the K-layer electron binding energy of an atom. Due to the photoabsorption of photons, there is a very large jump in the attenuation coefficient at the K-edge energy of the atom. In K-edge imaging, there may usually exist one or more materials with high atomic number, which may be called contrast agents. Contrast agent materials commonly used at present mainly include iodine (I), barium (Ba), gadolinium (Gd) and gold (Au). By setting the K-edge of the contrast agent element as the threshold for the PCD, the K-edge imaging may be used to identify and quantify the contrast agent material.

K-edge imaging is an imaging technique with the presence of contrast agents, and there are two main implementations currently:

The first implementation is the image subtraction method, which reconstructs the images on both sides of the K-edge energy of the contrast agent, respectively. Because of a large difference in the linear attenuation coefficients of the contrast agent materials on both sides of the K-edge energy, while the difference in the linear attenuation coefficients of other materials is not obvious, thus after the subtraction, the area of the contrast agent material may be highlighted very obviously, but the area of the background material is weakened.

The second implementation is the decomposition method, which performs a double-effect decomposition or a base material decomposition on the linear attenuation coefficient, and adds a linear attenuation coefficient of a contrast agent material as a basis function, and then uses a decomposition method such as the maximum likelihood method and the like, to obtain a spatial distribution of the basis function corresponding to the contrast agent material. Since the image subtraction method may cause a large noise, the decomposition method is used in embodiments of the present disclosure for threshold optimization.

For a better understanding of the present disclosure, a method, apparatus, device and medium for optimizing thresholds based on K-edge imaging according to embodiments of the present disclosure will be described in detail below with reference to the accompanying drawings. Please note that these embodiments are not intended to limit the scope of the present disclosure.

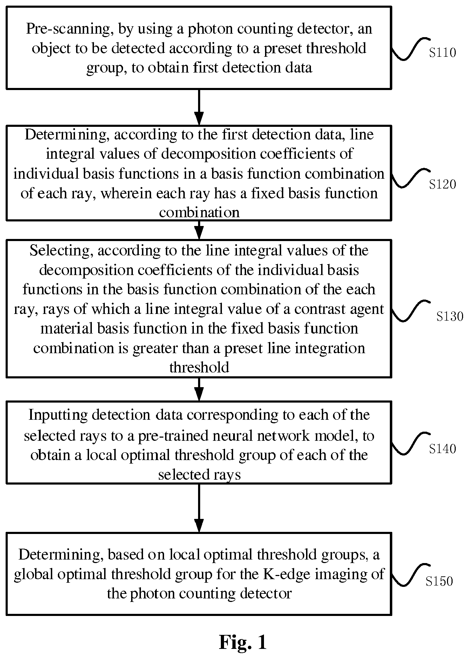

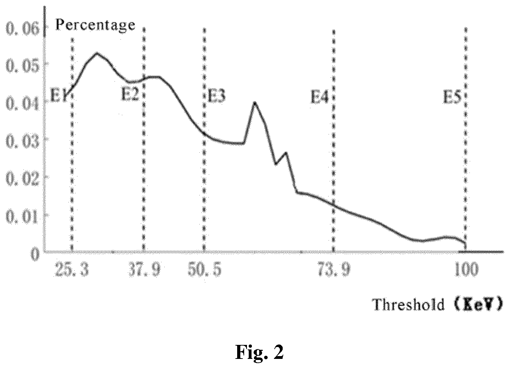

Fig. 1 is a flow chart showing a method for optimizing thresholds based on K-edge imaging according to an embodiment of the present disclosure. Fig. 2 is a schematic diagram showing a preset threshold group in an embodiment of the present disclosure.

As shown in Fig. 1, the method for optimizing thresholds based on K-edge imaging according to an embodiment of the present disclosure may include the following steps.

In step S110, pre-scanning, by using a PCD, an object to be detected according to a preset threshold group, to obtain first detection data.

In this step, for example, a tube voltage of an X-ray machine may be set as 80 kV peak (kVp), 90 kVp, and 100 kVp, respectively, and the object to be detected may scanned using the PCD to obtain the first detection data.

As shown in Fig. 2, the abscissa is the preset threshold group (unit: electron volt keV), and the ordinate is a percentage of the number of photons to the total number of photons under this preset threshold group. The preset threshold group may set the scan as E1, E2, E3, E4 and E5 from low to high. The preset threshold group may also be other forms of combination, which is not limited herein.

In the above embodiment, E1 = 25.3 keV, E2 = 37.9 keV, E3 = 50.5 keV, E4 = 73.9 keV, and E5 = 100 keV. E2 and E4 may be selected using the energy equipartition method. E1, E3, and E5 may be fixed thresholds, while E2 and E4 may be thresholds to be optimized and may be divided into two groups, for example, E1 and E2, E3 and E4, and alternatively, may be divided into a plurality of groups, which is not limited herein.

E1 and E2 may be inputted to the PCD to obtain a first set of detection data; and E3 and E4 may be inputted to the PCD to obtain a second set of detection data. The first and second set of detection data may constitute the first detection data.

In step S120, determining, according to the first detection data, line integral values of decomposition coefficients of individual basis functions in a basis function combination of each ray, wherein each ray has a fixed basis function combination.

In this step, a pre-processing algorithm based on a maximum likelihood method may be generally used. The pre-processing algorithm may first decompose the first detection data, and then reconstruct the decomposed data using a reconstructing algorithm. For the PCD, considering a response of the PCD, the Beer's law may be expressed by the following equation (1):

$$I = \int_{E_i}^{E_{i+1}} dE' \int_0^{+\infty} I_0(E) \exp\left(-\int_L \mu(\vec{r},E) d\vec{r}\right) h(E',E) dE$$

(1)

wherein, $h(E', E)$ is a response function of the PCD, $I$ is the number of photons after passing through the object to be detected, $I_0$ is the initial number of photons before passing through the object to be detected, the integral path L is a path of the X-ray passing through the object to be detected, $\mu(\vec{r},E)$ is a liner attenuation coefficient of the object to be detected at the position $\vec{r}$ under the threshold $E$, and $i$ is the number of energy windows.

No matter whether a base effect decomposition or a base material decomposition is performed on the first detection data, for the attenuation coefficient after the decomposition, the first detection data may be decomposed into a function related only to the path and a function related only to the energy.

Defining an integral of the function related only to the path in the first detection data, as equation (2):

$$\int a_\tau(\vec{r}) d\vec{r} \equiv A_\tau$$

(2)

wherein, $a_\tau(\vec{r})$ is a distribution of the basis function in the object to be detected, $A_\tau$ is the line integral value, and $\tau$ is the type of the basis function.

According to equations (1)-(2), the following equation may be obtained:

$$\begin{aligned}
\overline{n}_i &= \int_{E_i}^{E_{i+1}} dE' \int_0^{+\infty} I_0(E) \exp\left(-\sum_{\tau=1}^n A_\tau \phi_\tau(E)\right) h(E',E) dE \\
&= \int_0^{+\infty} I_0(E) \exp\left(-\sum_{\tau=1}^n A_\tau \phi_\tau(E)\right) dE \int_{E_i}^{E_{i+1}} h(E',E) dE' \\
&= \int_0^{+\infty} I_0(E) \exp\left(-\sum_{\tau=1}^n A_\tau \phi_\tau(E)\right) \mathrm{H}(i,E) dE
\end{aligned}$$

(3)

wherein,

$$H(i,E) \equiv \int_{E_i}^{E_{i+1}} h(E',E) dE'$$

(4)

wherein, $\phi_\tau(E)$ is the basis function, $\overline{n}_i$ is an average of the number of photons in the $i^{th}$ energy window, and H(i,E) is an integral of the response function $h(E',E)$ of the PCD.

By setting a probability model of the number of photons detected by each energy window of the PCD, the probability of the entire detecting process may be obtained.

For example, it may be assumed that the counts of each energy window of the PCD obey the Poisson distribution, then the probability of the number of photons detected by each energy window may be expressed as equation (5):

$$\Pr(n_1,\cdots,n_{N_E}) = \prod_{i=1}^{N_E} \frac{\overline{n}_i^{\,n_i}}{n_i!} e^{-\overline{n}_i} \tag{5}$$

wherein, $n_1,\cdots,n_{N_E}$ is the number of photons detected by each energy window, and $N_E$ is the number of the energy windows.

Taking a negative logarithm of equation (5) and omitting the constant term, the following equation (6) may be obtained:

$$\Phi(A_1,\cdots,A_\Gamma) = \sum_{i=1}^{N_E}(\overline{n}_i - n_i \ln \overline{n}_i)$$

$$= \sum_{i=1}^{N_E}\left[\int_0^{+\infty} I_0(E)e^{-\sum_{\tau=1}^{\Gamma} A_\tau f_\tau(E)} H(i,E)\mathrm{d}E - n_i \ln \int_0^{+\infty} I_0(E)e^{-\sum_{\tau=1}^{\Gamma} A_\tau f_\tau(E)} H(i,E)\mathrm{d}E \right] \tag{6}$$

wherein, $\Gamma$ is the number of the basis functions.

Then the maximum likelihood method may transform equation (6) to solve the following equation (7):

$$(\hat{A}_1,...,\hat{A}_\Gamma) = \arg\min_{A_\tau} \Phi(A_1,\cdots,A_\Gamma) \tag{7}$$

To solve equation (7), the commonly used solution is the Newton iteration method. If letting:

$$T_\tau \equiv \frac{\partial \Phi}{\partial A_\tau} = \sum_{i=1}^{N_E}\frac{\partial \overline{n}_i}{\partial A_\tau} - \frac{n_i}{\overline{n}_i}\frac{\partial \overline{n}_i}{\partial A_\tau} \tag{8}$$

Then the Newton iteration method may update equation (7) as the following equation (9):

$$\begin{bmatrix} A_1^{k+1} \\ \vdots \\ A_\Gamma^{k+1} \end{bmatrix} = \begin{bmatrix} A_1^{k} \\ \vdots \\ A_\tau^{k} \end{bmatrix} - \begin{bmatrix} \dfrac{\partial T_1}{\partial A_1} & \cdots & \dfrac{\partial T_1}{\partial A_\Gamma} \\ \vdots & \ddots & \vdots \\ \dfrac{\partial T_\Gamma}{\partial A_1} & \cdots & \dfrac{\partial T_\Gamma}{\partial A_\Gamma} \end{bmatrix}_{A_\tau^k}^{-1} \begin{bmatrix} \dfrac{\partial \Phi}{\partial A_1} \\ \vdots \\ \dfrac{\partial \Phi}{\partial A_\Gamma} \end{bmatrix}_{A_\tau^k} \tag{9}$$

wherein, k is the number of iterations.

In step S130, selecting, according to the line integral values of the decomposition coefficients of the individual basis functions in the basis function combination of the each ray, rays of which a line integral value of a contrast agent material basis function in the fixed basis function combination is greater than a preset line integration threshold.

In this step, since the region containing the contrast agent material basis function may be not the entire motif region, it is necessary to eliminate some rays having no or few contrast agent material basis function. Thus, a selecting threshold equation (10) may be set as:

$$\varepsilon = -\min\left(A_T\right) \qquad (10)$$

wherein, $A_T$ is a line integral value of the contrast agent material basis function, and $\varepsilon$ is the preset line integral threshold.

Note that in the absence of noise, the line integral value of the contrast agent material basis function that has been decomposed should be non-negative, but in practice, a negative value may occur due to the influence of noise.

In step S140, inputting detection data corresponding to each of the selected rays to a pre-trained neural network model, to obtain a local optimal threshold group of each of the selected rays.

In this step, the detection data corresponding to the above described rays for which $A_T > \varepsilon$ may be inputted to the pre-trained neural network model, to obtain the local optimal threshold group of the corresponding each ray.

In step S150, determining, based on local optimal threshold groups, a global optimal threshold group for K-edge imaging of the PCD.

In this step, the determined global optimal threshold group may be E2 = 34.3 keV and E4 = 70.3 keV.

With the method for optimizing thresholds according to embodiments of the present disclosure, it may be possible to roughly estimate an internal structure of the object to be detected using a low dose pre-scan form, thereby reducing the damage to the object to be detected. Secondly, the global optimal threshold group may be obtained effectively for an unknown object to be detected. Further, the use of the pre-trained neural network module to obtain the local optimal threshold group may improve the efficiency significantly.

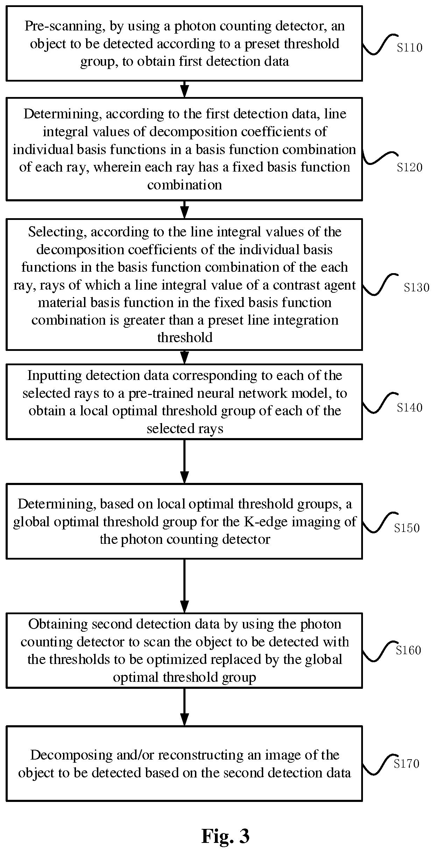

Fig. 3 is a detailed flowchart showing a method for optimizing thresholds according to another embodiment of the present disclosure, and the same or equivalent steps of Fig. 3 with Fig. 1 may use the same reference numerals.

As shown in Fig. 3, the method for optimizing thresholds in Fig. 3 may be substantially the same as the method for optimizing thresholds in Fig. 1, except that the method for optimizing thresholds in Fig. 3 may further include the following steps.

In step S160, obtaining second detection data by using the PCD to scan the object to be detected with the thresholds to be optimized replaced by the global optimal threshold group.

In this step, the thresholds may be set as E1 = 25.3 keV, E2 = 34.3 keV, E3 = 50.5 keV, E4 = 70.3 keV, and E5 = 100 keV. These five thresholds may be divided into two groups, for example, E1 and E2, E3 and E4, and alternatively, may be divided into a plurality of groups, which is not limited herein.

The scanning steps in this embodiment may be the same as the pre-scanning steps in the above embodiments, and will not be described in detail herein. References may be made to the above embodiments.

In step S170, decomposing and/or reconstructing an image of the object to be detected based on the second detection data.

In this step, the decomposition may be achieved utilizing the maximum likelihood method of equations (5)-(9) in the above embodiments, and the reconstruction of the distribution of the basis function solved above may be achieved using a common used reconstructing method such as the filtered back projection algorithm (FBP).

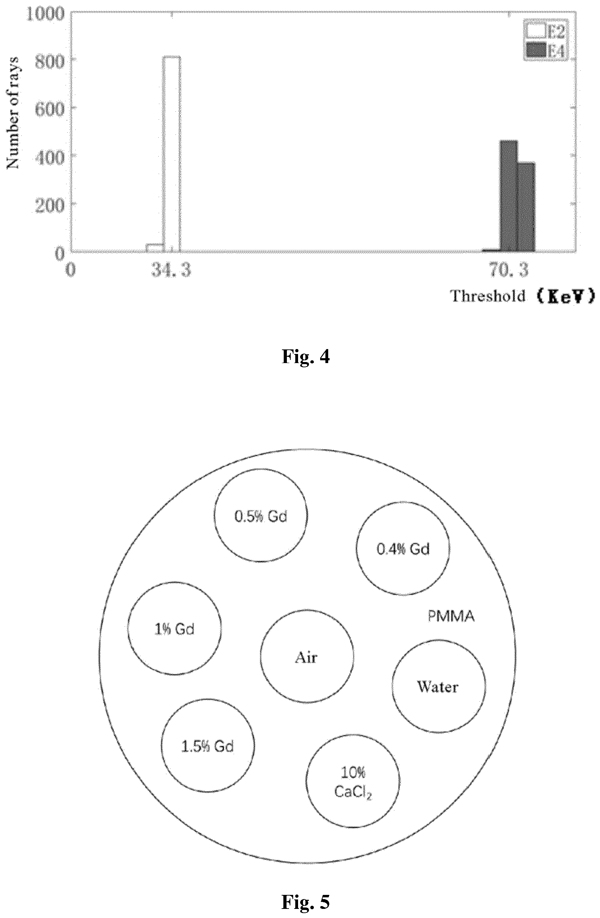

Fig. 4 is a schematic diagram showing a statistical local optimal threshold group in an embodiment of the present disclosure.

In one embodiment, the step S150 may include:
counting a number of rays for each of the local optimal threshold groups, and using a local optimal threshold group corresponding to a largest number of rays as the global optimal threshold group.

It may be understood that, as shown in Fig. 4, the ordinate is the number of rays, and the abscissa is the local optimal threshold group. Both E2 and E4 may be the thresholds to be optimized. The white bar may represent E2, the black bar may represent E4, the abscissa may indicate the appearance range of the local optimal threshold of E2 and E4, and the ordinate may indicate the number of rays present.

The number of local optimal threshold groups of all rays may be counted to find the threshold group with the largest number. This threshold group may be set as the global optimal threshold group, i.e., E2 and E4 of the threshold group with the largest number. Therefore, the global threshold group should be selected as E2 = 34.3 keV and E4 = 70.3 keV.

In one embodiment, the pre-scanning may include: a sparse angle scan and/or a low dose scan.

It should be appreciated that CT scans, especially K-edge imaging, may be usually used in medical applications. For patients, more scans may cause more radiations.

Therefore, scanning the object to be detected two times with normal dose to obtain the line integral values of the decomposition coefficients of each ray may be not practical, however, it may be necessary to obtain the internal structure of the object to be detected. Thus, a low dose pre-scan may be performed on the object to be detected. Although this low dose pre-scan may cause errors in an estimation of the line integral values of the decomposition coefficients of the object to be detected, the influence of the errors on the subsequent threshold optimization may be relatively small. Therefore, the low dose pre-scan may be an acceptable way to obtain the internal structure of the object to be detected.

In addition to the low dose scan, a sparse angle scan, or a combination of the low dose scan and the sparse angle scan and the like may also be used.

In one embodiment, the basis function combination may include: a photoelectric coefficient basis function and a Compton coefficient basis function.

It should be appreciated that, after the internal structure of the object to be detected is obtained by the low dose pre-scan, if the basis function combination is different, the result of the decomposition may also be different.

Since the main purpose of embodiments of the present disclosure is the threshold optimization for K-edge imaging, thus it may be necessary to select one basis function as the linear attenuation coefficient of the contrast agent material. To further simplify the problem, the basis function combination may be fixed as the photoelectric coefficient, the Compton coefficient, and the linear attenuation coefficient of the contrast agent material. In this manner, when calculating a noise lower bound, only the noise lower bound of the linear attenuation coefficient of the contrast agent material may be calculated and optimized. Therefore, it may be possible to avoid the influence of the difference of the decomposition basis function selection, and thus only focus on the threshold optimization for K-edge imaging.

Fig. 5 is a structural schematic diagram showing a training motif used in an embodiment of the present disclosure.

In one embodiment, the pre-trained neural network model may be a model obtained by using the local optimal threshold group as an output of a neural network model, and using the detection data corresponding to each of the selected rays as an input of the neural network model. The local optimal threshold group may comprise thresholds for the K-edge imaging of the PCD determined by the enumeration method when the noise lower bound of the contrast agent material basis function is minimum, wherein the noise lower bound of the contrast agent material may be determined by the Cramer-Rao lower bound theory.

It should be understood that, for the training of the neural network model, the local optimal threshold group may be used as the input of the neural network model. For example, the local optimal threshold group should be E2 = 34.3 keV and E4 = 70.3 keV. Specifically, the thresholds for the K-edge imaging of the PCD when the noise lower bound of the contrast agent material basis function is minimum may be determined by the enumeration method, while the noise lower bound of the contrast agent material basis function may be determined by the Cramer-Rao lower bound (CRLB) theory.

First, by step S110, the tube voltage of the X-ray machine may be set as 80 kVp, 90 kVp, 100 kVp, respectively, and the training motif as shown in Fig. 5 may be scanned using the PCD to get a training data. The training motif may include: water, 0.5% Gd. 0.4% Gd, 1% Gd, 1.5% Gd, air, 10% $CaCl_2$, and polymethyl methacrylate PMMA.

As the motif may contain the contrast agent Gd, and the K-edge of Gd may be about 50 keV, the optimal thresholds may be determined by setting different thresholds. After obtaining the line integral value $(\hat{A}_1,...,\hat{A}_\Gamma)$ of each ray according to equations (1)-(9), the noise lower bound of $(\hat{A}_1,...,\hat{A}_\Gamma)$ may be calculated by the CRLB theory. The Fisher information of equation (6) may be rewrote as equation (11) in a matrix form:

$$F_{\tau_1,\tau_2} = \left\langle -\frac{\partial^2 \Phi}{\partial A_{\tau_1} \partial A_{\tau_2}} \right\rangle \Bigg|_{\hat{A}_{\tau_1},\hat{A}_{\tau_2}} \tag{11}$$

wherein $\langle \cdot \rangle$ indicates an averaging.

Further,

$$\frac{\partial^2 \Phi}{\partial A_{\tau_1} \partial A_{\tau_2}} = \sum_{i=1}^{N_E} \left( \frac{\partial^2 \overline{n}_i}{\partial A_{\tau_1} \partial A_{\tau_2}} + \frac{n_i}{\overline{n}_i^2} \frac{\partial \overline{n}_i}{\partial A_{\tau_1}} \frac{\partial \overline{n}_i}{\partial A_{\tau_2}} - \frac{n_i}{\overline{n}_i} \frac{\partial^2 \overline{n}_i}{\partial A_{\tau_1} \partial A_{\tau_2}} \right) \tag{12}$$

$$\langle n_i \rangle = \overline{n}_i$$

$$\tag{13}$$

By substituting equations (13) and (12) into equation (11), the following equation (14) may be obtained:

$$F_{\tau_1,\tau_2} = \sum_{i=1}^{N_E} \frac{1}{\overline{n}_i} \frac{\partial \overline{n}_i}{\partial A_{\tau_1}} \frac{\partial \overline{n}_i}{\partial A_{\tau_2}} \Bigg|_{\hat{A}_{\tau_1},\hat{A}_{\tau_2}} \qquad (14)$$

For $\hat{A}_\tau$, its noise lower bound may satisfy the following equation (15):

$$\sigma_\tau^2 \geq \left[ \mathbf{F}^{-1} \right]_{\tau,\tau} \qquad (15)$$

wherein, F is the Fisher information matrix of $\Phi(A_1,...,A_\Gamma)$ obtained by solving equation (11), and $[\cdot]_{\tau,\tau}$ represents the diagonal elements of a matrix.

$[F^{-1}]_{\tau,\tau}$ represents the element of the $\tau$-th row and $\tau$-th column of the inverse matrix of F.

By substituting equation (3) into equation (14), the Fisher information matrix may be expressed by the following equation (16): [00123]

$$F_{\tau_1,\tau_2} = \sum_{i=1}^{N_E} \frac{1}{\overline{n}_i} \frac{\partial \overline{n}_i}{\partial A_{\tau_1}} \frac{\partial \overline{n}_i}{\partial A_{\tau_2}} \Bigg|_{\hat{A}_{\tau_1},\hat{A}_{\tau_2}}$$

$$= \sum_{i=1}^{N_E} \frac{\int_0^{+\infty} I_0(E)\phi_{\tau_1}(E)\exp\left(-\sum_{\tau=1}^n A_\tau\phi_\tau(E)\right)\mathrm{H}(i,\mathrm{E})\mathrm{d}E \int_0^{+\infty} I_0(E)\phi_{\tau_2}(E)\exp\left(-\sum_{\tau=1}^n A_\tau\phi_\tau(E)\right)\mathrm{H}(i,\mathrm{E})\mathrm{d}E}{\int_0^{+\infty} I_0(E)\exp\left(-\sum_{\tau=1}^n A_\tau\phi_\tau(E)\right)\mathrm{H}(i,\mathrm{E})\mathrm{d}E} \Bigg|_{\hat{A}_{\tau_1},\hat{A}_{\tau_2}} \qquad (16)$$

wherein, $\phi_\tau(E)$ are the various basis functions.

The value of each element in the Fisher information matrix may be obtained by solving equation (16), so as to enumerate the noise lower bounds of this ray under all threshold combinations. Thus, the minimum noise lower bound and the threshold group corresponding to the minimum noise lower bound may be found, and this threshold group may be the local optimal threshold group on this ray.

Due to the large amounts of calculation for obtaining the local optimal threshold group of each ray by the enumerate method, the time required for the calculation may be very long. Thus, the enumerate method may need to be accelerated.

For example, the look-up table method may be commonly used, in which the local optimal threshold group of each ray may be outputted by inputting the detection data of the ray. However, from equations (15)-(16), the physical quantities associated with the local optimal threshold group may include: an energy spectrum $I_0(E)$ of the ray machine, a line integral value $A_\tau$ of the various basis function, the basis function $\phi_\tau(E)$, and H(i,E), wherein all the $I_0(E)$, $\phi_\tau(E)$, and H(i,E) are vectors. If all the $I_0(E)$, $\phi_\tau(E)$, and H(i,E) are used as inputs of the look-up table, then the inputs of the look-up table may be too much, resulting in a memory explosion. Thus, the look-up method may be not suitable to this problem. A neural network model may therefore be used to obtain the local optimal threshold group of the individual ray. The efficiency for obtaining the local optimal threshold group of the individual ray may be increased greatly.

Because of the need to obtain the local optimal threshold group by the neural network model, the inputs of the neural network model should contain all relevant factors on the ray; and the output should be the local optimal threshold group of this ray.

According to equation (16), the factors effecting the CRLB noise lower bound of a certain ray may include: the energy spectrum $I_0(E)$ of the ray machine, the various basis function $\phi_\tau(E)$, the line integral value $A_\tau$, H(i,E), and the various thresholds E. Due to the need to obtain the local optimal threshold group of a certain ray, all the other influence factors except the threshold E should be used as the input end of the neural network model.

In equation (16), the threshold E may exist only in the upper and lower limits of the integral of each item, while the input end of the neural network model may not be effected by the threshold E. Thus, the input end of the neural network model may be set as the portion inside the integral sign of each item in equation (16). The specific process may be as follows.

First, defining:

$$f(E) = \exp(-\sum_{\tau=1}^{3} A_\tau \phi_\tau(E)) \tag{17}$$

Substituting equation (17) into equation (16), the following equation (18) may be obtained:

$$D_0 = h \times (I_0 \; e \; f)$$
$$D_1 = h \times (I_0 \; e \; \Phi_1 \; e \; f)$$
$$D_2 = h \times (I_0 \; e \; \Phi_2 \; e \; f)$$
$$D_3 = h \times (I_0 \; e \; \Phi_3 \; e \; f) \tag{18}$$

wherein, $h_{i \times j}$ is a response function matrix of the calibrated PCD, $i$ is the number of thresholds recovered by the response function, j is number of thresholds obtained by sweeping the spectrum in actual experiments, and i and j may not need to be completely consistent in actual use. $I_{0\;j\times 1}$ is the original energy spectrum of the ray machine, i.e., a discrete representation of the $I_0(E)$ in equation (16); $\Phi_{\tau\;j\times 1}$ is an attenuation coefficient of the $\tau$-th basis function, i.e., a discrete representation of the $\phi_\tau(E)$ in equation (17); and $f_{j\times 1}$ is a discrete representation of the $f(E)$ defined by equation (17). In equation (18), $\times$ represents a matrix multiplication, and e represents an element multiplication from bit to bits

In the above embodiment, $D_0$, $D_1$, $D_2$, $D_3$ may be vectors of the same dimension,

$D_0$ may be a discrete representation of $I_0(E)\exp\left(-\sum_{\tau=1}^{n} A_\tau \phi_\tau(E)\right)H(i,E)$ in equation (16);

$D_1$ may be a discrete representation of $I_0(E)\phi_{\tau_1}(E)\exp\left(-\sum_{\tau=1}^{n} A_\tau \phi_\tau(E)\right)H(i,E)$ in equation (16);

$D_2$ may be a discrete representation of $I_0(E)\phi_{\tau_2}(E)\exp\left(-\sum_{\tau=1}^{n} A_\tau \phi_\tau(E)\right)H(i,E)$ in equation (16);

$D_3$ may be a discrete representation of $I_0(E)\phi_{\tau_3}(E)\exp\left(-\sum_{\tau=1}^{n} A_\tau \phi_\tau(E)\right)H(i,E)$ in equation (16).

After a respective normalization of the four vectors $D_0$, $D_1$, $D_2$, $D_3$, they may be arranged into a new vector in the order of $D_0$, $D_1$, $D_2$, $D_3$. The new vector may be used as the input of the neural network model, and the thresholds of the K-edge imaging of the PCD determined by the enumeration method when the noise lower bound of the contrast agent material basis function is minimum may be used as the output of the neural network model. Further, the error inverse propagation algorithm may be utilized to train the neuron parameters of the neural network model, to obtain a trained neural network model, i.e., the pre-trained neural network model.

The following is an instruction of the above described pre-trained neural network model.

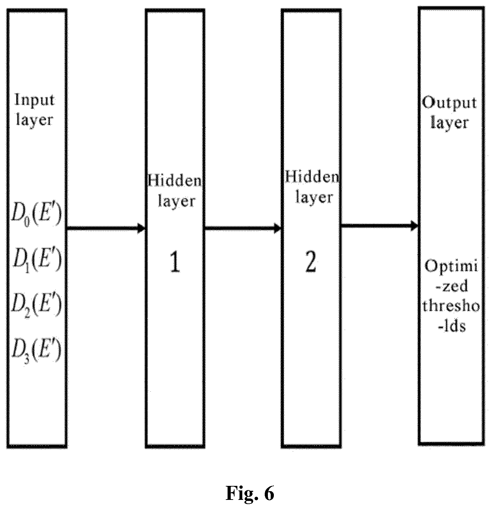

Fig. 6 is a structural schematic diagram showing the pre-trained neural network model in an embodiment of the present disclosure.

For the structure design of the neural network model, the input layer, output layer and hidden layer of the model may need be designed according to the actual problem. In embodiments of the present disclosure, the neural network model may use the BP fully connected neural network model. For the BP fully connected neural network model, the design of the input layer, output layer and hidden layer of the model may be the design of the number of neurons and the type of response functions of the model. In theory, the more the number of layers of the BP fully connected neural network model, the less the error of the training set of the BP fully connected neural network model. However, a large number of layers may increase the training difficulty of the BP fully connected neural network model and slow down the training. In addition, if the structure of the BP fully connected neural network model is too complicated, this may cause an over-fitting for the BP fully connected neural network model. That is, the fitting degree of the training set may be too strict, resulting in that the BP fully connected neural network model may be difficult to apply to other situations than the training set, and decreasing the generalization performance of the BP fully connected neural network model.

For continuous problems, a BP fully connected neural network model with a single hidden layer may be usually utilized. However, for discontinuous function problems, a BP fully connected neural network model with more than one hidden layer may be required. At present, there is not an acknowledged method for selecting the number of layers of the network, but it is default that a network with a single hidden layer may be utilized for continuous problems, and a network with a plurality of hidden layer may be utilized for discontinuous problems. The neurons in hidden layers may significantly affect the result of the BP fully connected neural network model. As the BP fully connected neural network model may learn relevant rules from sample data, if the number of nodes is too small, the learning degree of the BP fully connected neural network model for the training data may be insufficient, which may cause the data feature of the training data could not be obtained well. And if the number of nodes is too large, it may also cause an over-fitting of the BP fully connected neural network model. Thus, in the design of the BP fully connected neural network model, it may be necessary to select an appropriate number of neurons to make various performances of the BP fully connected neural network model to be better.

In the actual selecting procedure, as there is not a mature method for selecting the number of neurons at present, the trial and error method may be generally used. The starting point of the trial and error method may be determined by the following equation (19):

$$l < n - 1$$
$$l = \sqrt{m + n} + a \tag{19}$$

wherein, $l$ is the number of neurons in the hidden layer, $n$ is the number of neurons in the input layer, $m$ is the number of neurons in the output layer, and $a$ is an integer between 1 and 10. Equation (19) may be just the starting point of the trial and error method, and in the actual use, it may be necessary to try around the starting point to determine the number of neurons.

The problem of embodiments of the present disclosure is a discontinuous problem, thus the neural network model may need to utilize a multi-hidden layer structure. After experiment, a two hidden layer structure may be utilized. As shown in Fig. 6, the pre-trained neural network model may include: an input layer, a hidden layer 1, a hidden layer 2, and an output layer. The number of neurons in each hidden layer may be estimated firstly by equation (19), and then adjusted around the estimated value. In embodiments of the present disclosure, the utilized neural network model may be a BP neural network model with two hidden layers, wherein the number of neurons in the hidden layer 1 may be 25, and the number of neurons in the hidden layer 2 may be 40.

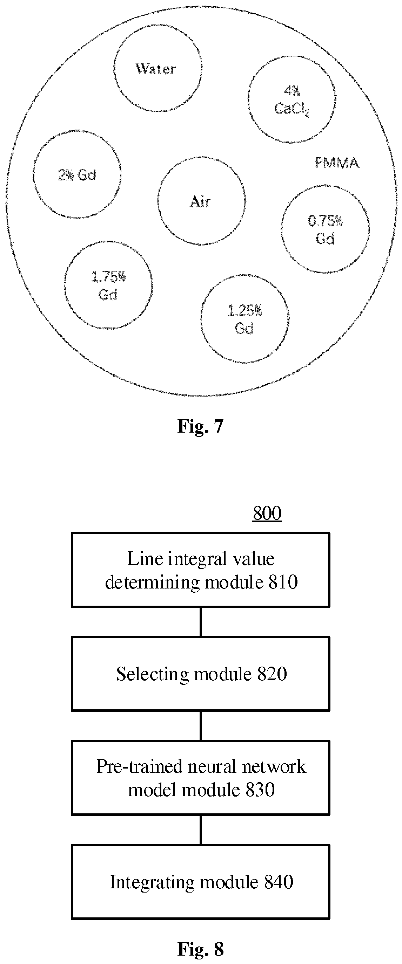

Fig. 7 is a structural schematic diagram showing a testing motif used in an embodiment of the present disclosure.

In actual experiments, as the energy step interval of the response function of the PCD is 1.8 KeV, thus one unit length may be defines as 1.8 KeV. To better evaluate the performance of the trained neural network model, the testing motif as shown in Fig. 7 may be used, which may include: water, 2% Gd, 1.75% Gd, 1.25% Gd, 0.75% Gd, air, 4% $CaCl_2$ and polymethyl methacrylate PMMA. The tube voltage of the X-ray machine may be set as 95 kVp. The threshold settings of the pre-scanning may be the same as that of step S110, to obtain the testing data of the pre-scanning under each threshold, and calculate line integral values of individual basis functions of each ray. The testing data of the selected ray may be input to the trained neural network model to obtain the local optimal threshold of each ray output by the network. To facilitate the express of the accuracy of the output of the neural network model, the error may be defined as: error=y - y, the unit of which is one unit length, wherein $\hat{y}$ is the local optimal threshold group output by the neural network model, and $y$ is the local optimal threshold group obtained by performing the enumeration method on the training data or testing data.

The apparatus for optimizing thresholds according to embodiments of the present disclosure will be described in detail below with reference to the accompanying drawings.

Fig. 8 is a structural schematic diagram showing an apparatus for optimizing thresholds based on K-edge imaging provided by an embodiment of the present disclosure. As shown in Fig. 8, the apparatus 800 for optimizing thresholds may include:

a line integral value determining module 810, a selecting module 820, a pre-trained neural network model module 830, and an integrating module840.

The line integral value determining module 810 may be configured to determine, according to a first detection data, line integral values of decomposition coefficients of individual basis functions in a basis function combination of each ray, wherein each ray may have a fixed basis function combination; and wherein, the first detection data may be obtained by using a PCD to pre-scan an object to be detected according to a preset threshold group.

The selecting module 820 may be configured to select, according to the line integral values of the decomposition coefficients of the individual basis functions in the basis function combination of the each ray, rays of which a line integral value of a contrast agent material basis function in the fixed basis function combination is greater than a preset line integration threshold.

The pre-trained neural network model module 830 may be configured to input detection data corresponding to each of the selected rays to a pre-trained neural network model, to obtain a local optimal threshold group of each of the selected rays.

The integrating module 840 may be configured to determine, based on local optimal threshold groups, a global optimal threshold group for K-edge imaging of the PCD.

With the apparatus for optimizing thresholds according to embodiments of the present disclosure, it may be possible to roughly estimate the internal structure of the object to be detected using a low dose pre-scan form, thereby reducing the damage to the object to be detected. Secondly, the global optimal threshold group may be obtained effectively for an unknown object to be detected. Further, the use of the pre-trained neural network module to obtain the local optimal threshold group may improve the efficiency significantly.

In one embodiment, the preset threshold group may include fixed thresholds and thresholds to be optimized, and the thresholds to be optimized may be obtained by an energy equipartition manner.

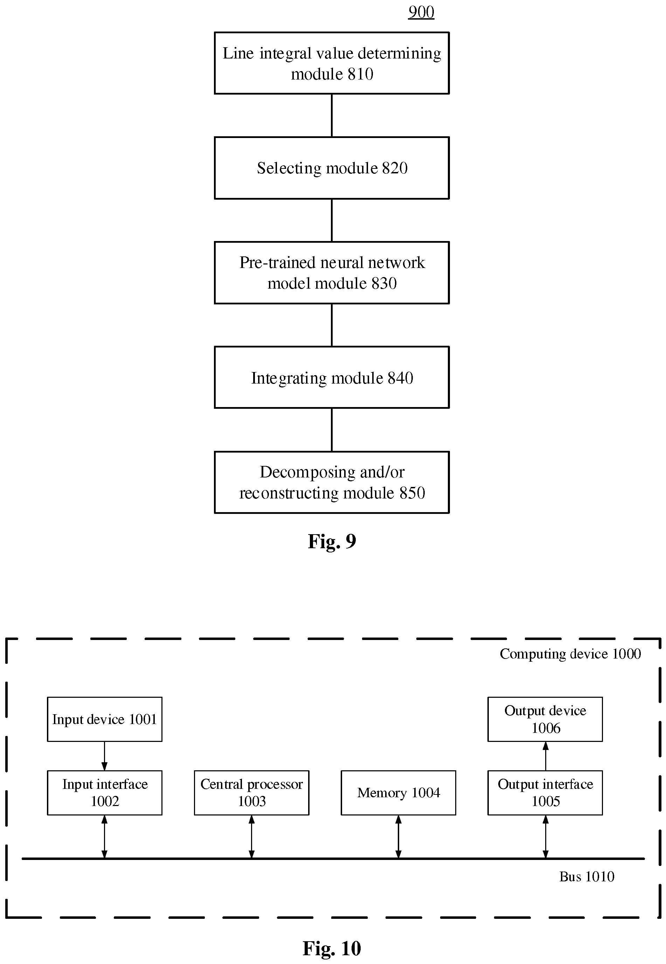

Fig. 9 is a structural schematic diagram showing an apparatus for optimizing thresholds according to another embodiment of the present disclosure, and the same or equivalent modules of Fig. 9 with Fig. 8 may use the same reference numerals.

As shown in Fig. 9, the apparatus 900 for optimizing thresholds may be substantially the same as the apparatus 800 for optimizing thresholds, except that the apparatus 900 for optimizing thresholds may further include:
a decomposing and/or reconstructing module 850 which may be configured to decompose and/or reconstruct an image of the object to be detected based on second detection data, wherein the second detection data may be obtained by using the PCD to scan the object to be detected with the thresholds to be optimized replaced by the global optimal threshold group.

In one embodiment, the integrating module 840 may be configured to count a number of rays for each of the local optimal threshold groups, and use a local optimal threshold group corresponding to a largest number as the global optimal threshold group.

In one embodiment, the pre-scanning may include: a sparse angle scan and/or a low dose scan.

In one embodiment, the line integral value determining module 810 may be configured to calculate the first detection data using a maximum likelihood decomposition method, to determine the line integral values of the decomposition coefficients of individual basis functions in the basis function combination of each ray.

In one embodiment, the basis function combination may include: a photoelectric coefficient basis function and a Compton coefficient basis function.

In one embodiment, the pre-trained neural network model may be obtained a model by using the local optimal threshold group as an output of a neural network model, and using the detection data corresponding to each of the selected rays as an input of the neural network model. The local optimal threshold group may comprise thresholds for the K-edge imaging of the PCD determined by an enumeration method when a noise lower bound of the contrast agent material basis function is minimum, wherein the noise lower bound of the contrast agent material basis function may be determined by the CRLB theory.

Other details of the apparatus for optimizing thresholds according to embodiments of the present disclosure may be similar to that of the above method for optimizing thresholds according to embodiments of the present disclosure described with reference to Fig. 1 to Fig. 9, which will not described herein.

Fig. 10 is a block diagram showing an exemplary hardware architecture of a computing device capable of implementing the method and apparatus for optimizing thresholds in accordance with embodiments of the present disclosure.

As shown in Fig. 10, the computing device 1000 may include: an input device 1001, an input interface 1002, a central processor 1003, a memory 1004, an output interface 1005, and an output device 1006. The input interface 1002, the central processor 1003, the memory 1004, the output interface 1005 may connect with each other by a bus 1010 The input device 1001 and the output device 1006 may connect with the bus 1010 through the input interface 1002 and the output interface 1005, respectively, and further with other components of the computing device 1000. Specifically, the input device 1001 may receive input information from the outside, and transmit the input information to the central processor 1003 through the input interface 1002; the central processor 1003 may process the input formation based on computer executable instructions stored in the memory 1004 to generate output information, and store the output information in the memory 1004 temporarily or permanently, and then transmit the output information to the output device 1006 through the output interface 1005; and the output device 1006 may output the output information to the outside of the computing device 1000 for use by the user.

In one embodiment, the computing device 1000 as shown in Fig. 10 may be implemented as a terminal device, which may include: a memory and a processor; the memory may be configured to store executable program codes; and the processor may be configured to read the executable program codes stored in the memory to perform the method for optimizing thresholds of the above embodiments.

The above described embodiments may be implemented in whole or in part by software, hardware, firmware, or any combination thereof. When implemented by software, the above described embodiments may be implemented in whole or in part in the form of computer program product or computer readable storage medium. The computer program product or computer readable storage medium may include one or more computer instructions. The computer program instructions, when loaded an executed by a computer, may product in whole or in part the processes or functions described in accordance with embodiments of the present disclosure. The computer may be a general purpose computer, a special purpose computer, a computer network, or other programmable devices. The computer instructions may be stored in a computer readable storage medium, or transferred from one computer readable storage medium to another one, for example, the computer instructions may be transmitted from a website site, a computer, a server or data center to another website site, computer, server or data center by wire (e.g., coaxial cable, fiber optic, digital subscriber line (DSL)) or wireless (e.g., infrared, wireless, microwave, etc.) means. The computer readable storage medium may be any available medium accessible to a computer, or a data storage device, such as a server, a date center, containing one or more available medium integration. The available medium may be a magnetic medium (e.g., a floppy disk, a hard disk, a magnetic tape), an optical medium (e.g., DVD), or a semiconductor (e.g., a Solid State Disk (SSD)), and the like.

It should be noted that the present disclosure is not limited to the specific configurations and processes described above and illustrated in the drawings. For simplicity, a detailed description of well-known methods are omitted herein. In the above embodiments, several specific steps have been described and illustrated as examples. However, the method of the present disclosure is not limited to the specific steps have been described and illustrated. Those skilled in the art may make various changes, modifications and additions, or change the order of the steps within the scope of the present disclosure.

The present disclosure may be implemented in other specific forms, without departing from the scope and essential characteristics thereof. Therefore, the preset embodiments are considered as illustrative and non-limiting in all respects. The scope of the present disclosure is defined by the appended claims rather than the above descriptions, and all changes falling within the meanings and equivalents of the claims are thus included in the scope of the present disclosure. Moreover, different technical features in different embodiments may be combined to achieve beneficial effects. Those skilled in the art may be able to understand and achieve embodiments of other changes of the disclosed embodiments on a basis of a study of the accompanying drawings, specification, and claims.

**Claims**

1. A method for optimizing thresholds based on K-edge imaging, comprising:

    pre-scanning (S110), by using a photon counting detector, an object to be detected according to a preset threshold group, to obtain first detection data;
    determining (S 120), according to the first detection data, line integral values of decomposition coefficients of individual basis functions in a basis function combination of each ray, wherein each ray has a fixed basis function combination;
    selecting (S 130), according to the line integral values of the decomposition coefficients of the individual basis functions in the basis function combination of the each ray, rays of which a line integral value of a contrast agent material basis function in the fixed basis function combination is greater than a preset line integration threshold;
    inputting (S140) detection data corresponding to each of the selected rays to a pre-trained neural network model, to obtain a local optimal threshold group of each of the selected rays; and
    determining (S 150), based on local optimal threshold groups, a global optimal threshold group for the K-edge imaging of the photon counting detector.

2. The method of claim 1, wherein the preset threshold group comprises fixed thresholds and thresholds to be optimized, and the thresholds to be optimized are obtained by an energy equipartition manner.

3. The method of claim 2, further comprising, after determining (S 150), based on the local optimal threshold groups, the global optimal threshold group for the K-edge imaging of the photon counting detector:

    obtaining (S160) second detection data by using the photon counting detector to scan the object to be detected with the thresholds to be optimized replaced by the global optimal threshold group; and
    decomposing and/or reconstructing (S170) an image of the object to be detected based on the second detection data.

**4.** The method of claim 1, wherein determining (S150), based on the local optimal threshold groups, the global optimal threshold group for the K-edge imaging of the photon counting detector comprises:
counting a number of rays for each of the local optimal threshold groups, and using a local optimal threshold group corresponding to a largest number of rays as the global optimal threshold group.

**5.** The method of claim 1, wherein determining (S 120), according to the first detection data, the line integral values of the decomposition coefficients of the individual basis functions in the basis function combination of each ray comprises:
calculating the first detection data using a maximum likelihood decomposition method, to determine the line integral values of the decomposition coefficients of the individual basis functions in the basis function combination of each ray.

**6.** The method of claim 1, wherein the basis function combination comprises: a photoelectric coefficient basis function and a Compton coefficient basis function.

**7.** The method of claim 1, wherein the pre-trained neural network model is a model obtained by using the local optimal threshold group as an output of a neural network model and using the detection data corresponding to each of the selected rays as an input of the neural network model,
wherein the local optimal threshold group comprises thresholds for the K-edge imaging of the photon counting detector determined by an enumeration method when a noise lower bound of the contrast agent material basis function is minimum, wherein the noise lower bound of the contrast agent material basis function is determined by a Cramer-Rao lower bound theory.

**8.** An apparatus (800, 900) for optimizing thresholds based on K-edge imaging, comprising:

a line integral value determining module (810), a selecting module (820), a pre-trained neural network model module (830) and an integrating module (840);
the line integral value determining module (810) is configured to determine, according to first detection data, line integral values of decomposition coefficients of individual basis functions in a basis function combination of each ray, wherein each ray has a fixed basis function combination; and wherein, the first detection data is obtained by using a photon counting detector to pre-scan an object to be detected according to a preset threshold group;
the selecting module (820) is configured to select, according to the line integral values of the decomposition coefficients of the individual basis functions in the basis function combination of the each ray, rays of which a line integral value of a contrast agent material basis function in the fixed basis function combination is greater than a preset line integration threshold;
the pre-trained neural network model module (830) is configured to input detection data corresponding to each of the selected rays to a pre-trained neural network model, to obtain a local optimal threshold group of each of the selected rays; and
the integrating module (840) is configured to determine, based on local optimal threshold groups, a global optimal threshold group for the K-edge imaging of the photon counting detector.

**9.** The apparatus (800, 900) of claim 8, wherein the preset threshold group comprises fixed thresholds and thresholds to be optimized, and the thresholds to be optimized are obtained by an energy equipartition manner.

**10.** The apparatus (800, 900) of claim 9, further comprising:

a decomposing and/or reconstructing module (850),
the decomposing and/or reconstructing module (850) is configured to decompose and/or reconstruct an image of the object to be detected based on second detection data, wherein the second detection data is obtained by using the photon counting detector to scan the object to be detected with the thresholds to be optimized replaced by the global optimal threshold group.

**11.** The apparatus (800, 900) of claim 8, wherein the integrating module (840) is further configured to:
count a number of rays for each of the local optimal threshold groups, and use a local optimal threshold group corresponding to a largest number of rays as the global optimal threshold group.

**12.** The apparatus (800, 900) of claim 8, wherein the line integral value determining module (810) is further configured to:
calculate the first detection data using a maximum likelihood decomposition method, to determine the line integral values of the decomposition coefficients of individual basis functions in the basis function combination of each ray.

13. The apparatus (800, 900) of claim 8, wherein the basis function combination comprises: a photoelectric coefficient basis function and a Compton coefficient basis function.

14. The apparatus (800, 900) of claim 8, wherein the pre-trained neural network model is a model obtained by using the local optimal threshold group as an output of a neural network model and using the detection data corresponding to each of the selected rays as an input of the neural network model, wherein the local optimal threshold group comprises thresholds for the K-edge imaging of the photon counting detector determined by an enumeration method when a noise lower bound of the contrast agent material basis function is minimum, wherein the noise lower bound of the contrast agent material basis function is determined by a Cramer-Rao lower bound theory.

15. A computer storage medium comprising instructions which, when executed on a computer, cause the computer to perform the method for optimizing thresholds of any of claims 1-7.


**Patentansprüche**

1. Verfahren zum Optimieren von Schwellenwerten basierend auf K-Kanten-Bildgebung, umfassend:

   Vorscannen (S110) eines zu detektierenden Objekts gemäß einer voreingestellten Schwellenwertgruppe unter Verwendung eines Photonenzähldetektors, um erste Detektionsdaten zu erhalten;
   Bestimmen (S120) gemäß den ersten Detektionsdaten von Linienintegralwerten von Zerlegungskoeffizienten der individuellen Basisfunktionen in einer Basisfunktionskombination jedes Strahls, wobei jeder Strahl eine feste Basisfunktionskombination aufweist;
   Auswählen (S130) gemäß den Linienintegralwerten der Zerlegungskoeffizienten der individuellen Basisfunktionen in der Basisfunktionskombination jedes Strahls von Strahlen, von denen ein Linienintegralwert einer Kontrastmittelmaterial-Basisfunktion in der festen Basisfunktionskombination größer als ein voreingestellter Linienintegrationsschwellenwert ist;
   Eingeben (S140) von Detektionsdaten, die jedem der ausgewählten Strahlen entsprechen, in ein vorab trainiertes neuronales Netzwerkmodell, um eine lokale optimale Schwellenwertgruppe von jedem der ausgewählten Strahlen zu erhalten; und
   Bestimmen (S150), basierend auf lokalen optimalen Schwellenwertgruppen, einer globalen optimalen Schwellenwertgruppe für die K-Kanten-Bildgebung des Photonenzähldetektors.

2. Verfahren nach Anspruch 1, wobei die voreingestellte Schwellenwertgruppe feste Schwellenwerten und zu optimierende Schwellenwerten umfasst und die zu optimierenden Schwellenwerten durch eine Energiegleichverteilungsweise erhalten werden.

3. Verfahren nach Anspruch 2, ferner umfassend, nach dem Bestimmen (S150), basierend auf den lokalen optimalen Schwellenwertgruppen, der globalen optimalen Schwellenwertgruppe für die K-Kanten-Bildgebung des Photonenzähldetektors:

   Erhalten (S160) zweiter Detektionsdaten durch Verwendung des Photonenzähldetektors zum Abtasten des zu detektierenden Objekts, wobei die zu optimierenden Schwellenwerte durch die globale optimale Schwellenwertgruppe ersetzt werden; und
   Zerlegen und/oder Rekonstruieren (S170) eines Bildes des zu detektierenden Objekts basierend auf den zweiten Detektionsdaten.

4. Verfahren nach Anspruch 1, wobei das Bestimmen (S150), basierend auf den lokalen optimalen Schwellenwertgruppen, der globalen optimalen Schwellenwertgruppe für die K-Kanten-Bildgebung des Photonenzähldetektors umfassend:
   Zählen einer Anzahl von Strahlen für jede der lokalen optimalen Schwellenwertgruppen und Verwenden einer lokalen optimalen Schwellenwertgruppe, die einer größten Anzahl von Strahlen entspricht, als die globale optimale Schwellenwertgruppe.

5. Verfahren nach Anspruch 1, wobei das Bestimmen (S120) gemäß den ersten Detektionsdaten von Linienintegralwerten von Zerlegungskoeffizienten der individuellen Basisfunktionen in einer Basisfunktionskombination jedes Strahls umfassend:
Berechnen der ersten Detektionsdaten unter Verwendung eines Maximum-Likelihood-Zerlegungsverfahrens, um die Linienintegralwerte der Zerlegungskoeffizienten der individuellen Basisfunktionen in der Basisfunktionskombination jedes Strahls zu bestimmen.

6. Verfahren nach Anspruch 1, wobei die Basisfunktionskombination umfassend: eine photoelektrische Koeffizienten-Basisfunktion und eine Compton-Koeffizienten-Basisfunktion.

7. Verfahren nach Anspruch 1, wobei das vorab trainierte neuronale Netzwerkmodell ein Modell ist, das durch Verwenden der lokalen optimalen Schwellenwertgruppe als eine Ausgabe eines neuronalen Netzwerkmodells und Verwenden der Detektionsdaten, die jedem der ausgewählten Strahlen entsprechen, als eine Eingabe des neuronalen Netzwerkmodells erhalten wird,
wobei die lokale optimale Schwellenwertgruppe Schwellenwerte für die K-Kanten-Bildgebung des Photonenzähldetektors umfasst, die durch ein Aufzählungsverfahren bestimmt werden, wenn eine untere Rauschgrenze der Kontrastmittelbasisfunktion minimal ist, wobei die untere Rauschgrenze der Kontrastmittelmaterial-Basisfunktion durch eine Cramer-Rao-Untergrenzentheorie bestimmt wird.

8. Vorrichtung (800, 900) zum Optimieren von Schwellenwerten basierend auf einer K-Kanten-Bildgebung, umfassend:

ein Linienintegralwert-Bestimmungsmodul (810), ein Auswahlmodul (820), ein vortrainiertes neuronales Netzwerkmodellmodul (830) und ein Integrationsmodul (840);
das Linienintegralwert-Bestimmungsmodul (810) dazu konfiguriert ist, gemäß ersten Detektionsdaten Linienintegralwerte von Zerlegungskoeffizienten individueller Basisfunktionen in einer Basisfunktionskombination jedes Strahls zu bestimmen, wobei jeder Strahl eine feste Basisfunktionskombination hat; und wobei die ersten Detektionsdaten erhalten werden, indem ein Photonenzähldetektor verwendet wird, um ein zu detektierendes Objekt gemäß einer voreingestellten Schwellenwertgruppe vorab abzutasten;
das Auswahlmodul (820) so konfiguriert ist, dass es gemäß den Linienintegralwerten der Zerlegungskoeffizienten der individuellen Basisfunktionen in der Basisfunktionskombination jedes Strahls Strahlen auswählt, von denen ein Linienintegralwert einer Kontrastmittelmaterial-Basisfunktion in der festen Basisfunktionskombination größer als ein voreingestellter Zeilenintegrationsschwellenwert ist;
das vortrainierte neuronale Netzwerkmodellmodul (830) so konfiguriert ist, dass es Detektionsdaten, die jedem der ausgewählten Strahlen entsprechen, in ein vortrainiertes neuronales Netzwerkmodell eingibt, um eine lokale optimale Schwellenwertgruppe von jedem der ausgewählten Strahlen zu erhalten; und
das Integrationsmodul (840) dazu konfiguriert ist, basierend auf lokalen optimalen Schwellenwertgruppen eine globale optimale Schwellenwertgruppe für die K-Kanten-Bildgebung des Photonenzähldetektors zu bestimmen.

9. Vorrichtung (800, 900) nach Anspruch 8, wobei die voreingestellte Schwellengruppe feste Schwellenwerten und zu optimierende Schwellenwerten umfasst und die zu optimierenden Schwellenwerten durch eine Energiegleichverteilungsweise erhalten werden.

10. Vorrichtung (800, 900) nach Anspruch 9, ferner umfassend:

ein Zerlegungs- und/oder Rekonstruktionsmodul (850),
das Zerlegungs- und/oder Rekonstruktionsmodul (850) dazu konfiguriert ist, ein Bild des zu detektierenden Objekts basierend auf zweiten Detektionsdaten zu zerlegen und/oder zu rekonstruieren, wobei die zweiten Detektionsdaten durch Verwendung des Photonenzähldetektors zum Scannen des zu detektierenden Objekts erhalten werden, wobei die zu optimierenden Schwellenwerte durch die globale optimale Schwellenwertgruppe ersetzt werden.

11. Vorrichtung (800, 900) nach Anspruch 8, wobei das Integrationsmodul (840) ferner konfiguriert ist zum:
Zählen einer Anzahl von Strahlen für jede der lokalen optimalen Schwellenwertgruppen und Verwenden einer lokalen optimalen Schwellenwertgruppe, die einer größten Anzahl von Strahlen entspricht, als die globale optimale Schwellenwertgruppe.

12. Vorrichtung (800, 900) nach Anspruch 8, wobei das Linienintegralwert-Bestimmungsmodul (810) ferner konfiguriert ist zum:
Berechnen der ersten Detektionsdaten unter Verwendung eines Maximum-Likelihood-Zerlegungsverfahrens, um die Linienintegralwerte der Zerlegungskoeffizienten individueller Basisfunktionen in der Basisfunktionskombination jedes Strahls zu bestimmen.

13. Vorrichtung (800, 900) nach Anspruch 8, wobei die Basisfunktionskombination umfassend: eine photoelektrische Koeffizienten-Basisfunktion und eine Compton-Koeffizienten-Basisfunktion.

14. Vorrichtung (800, 900) nach Anspruch 8, wobei das vorab trainierte neuronale Netzwerkmodell ein Modell ist, das durch Verwenden der lokalen optimalen Schwellenwertgruppe als eine Ausgabe eines neuronalen Netzwerkmodells und Verwenden der Detektionsdaten, die jedem der ausgewählten Strahlen entsprechen, als eine Eingabe des neuronalen Netzwerkmodells erhalten wird, wobei die lokale optimale Schwellenwertgruppe Schwellenwerte für die K-Kanten-Bildgebung des Photonenzähldetektors umfasst, die durch ein Aufzählungsverfahren bestimmt werden, wenn eine untere Rauschgrenze der Kontrastmittelbasisfunktion minimal ist, wobei die untere Rauschgrenze der Kontrastmittelmaterial-Basisfunktion durch eine Cramer-Rao-Untergrenzentheorie bestimmt wird.

15. Computerspeichermedium mit Befehlen, die, wenn sie auf einem Computer ausgeführt werden, den Computer veranlassen, das Verfahren zum Optimieren von Schwellenwerten nach einem der Ansprüche 1-7 durchzuführen.


**Revendications**

1. Procédé d'optimisation de seuils basé sur l'imagerie K-edge, comprenant :

   pré-balayer (S110), par un détecteur de comptage photonique, un objet à détecter selon un groupe de seuils prédéfinis, de sorte d'obtenir des premières données de détection ;
   déterminer (S120), en fonction des premières données de détection, des valeurs d'intégration linaire de coefficients de décomposition de fonctions de base individuelles dans une combinaison de fonctions de base de chaque rayon, dans lequel chaque rayon correspond à une combinaison de fonctions de base fixe ;
   sélectionner (S130), en fonction des valeurs d'intégration linaire des coefficients de décomposition des fonctions de base individuelles dans la combinaison de fonctions de base de chaque rayon, des rayons dont une valeur d'intégration linaire de la fonction de base d'un matériau d'agent de contraste dans la combinaison de fonctions de base fixe est supérieure à un seuil d'intégration linaire prédéfini ;
   entrer (S 140) des données de détection correspondant à chacun des rayons sélectionnés dans un modèle de réseau de neurones pré-entraîné, de sorte d'obtenir un groupe de seuils optimal local de chacun des rayons sélectionnés ; et
   déterminer (S150), sur la base des groupes de seuils optimaux locaux, un groupe de seuils optimal global pour une imagerie K-edge du détecteur de comptage photonique.

2. Procédé selon la revendication 1, dans lequel le groupe de seuils prédéfinis comprend des seuils fixes et des seuils à optimiser, les seuils à optimiser étant obtenus par une méthode d'équipartition énergétique.

3. Procédé selon la revendication 2, comprenant en outre, après l'étape de déterminer (S150), sur la base des groupes de seuils optimaux locaux, le groupe de seuils optimal global pour l'imagerie K-edge du détecteur de comptage photonique :

   acquérir (S 160), par le détecteur de comptage photonique, des deuxièmes données de détection, pour balayer l'objet à détecter avec les seuils à optimiser remplacés par le groupe de seuil optimal global ; et
   décomposer et/ou reconstruire (S170) une image de l'objet à détecter sur la base des deuxièmes données de détection.

4. Procédé selon la revendication 1, dans lequel, l'étape de déterminer (S150), sur la base des groupes de seuils optimaux locaux, le groupe de seuils optimal global pour l'imagerie K-edge du détecteur de comptage photonique comprend :
   compter un nombre de rayons pour chacun des groupes de seuils optimaux locaux, et utiliser un groupe de seuils optimal local correspondant au plus grand nombre de rayons comme groupe de seuils optimal global.

5. Procédé selon la revendication 1, dans lequel l'étape de déterminer (S120), en fonction des premières données de détection, les valeurs d'intégration linaire des coefficients de décomposition des fonctions de base individuelles dans la combinaison de fonctions de base de chaque rayon comprend :
   calculer les premières données de détection par une méthode de décomposition de vraisemblance maximale, de sorte de déterminer les valeurs d'intégration linaire des coefficients de décomposition des fonctions de base individuelles dans la combinaison de fonctions de base de chaque rayon.

6. Procédé selon la revendication 1, dans lequel la combinaison de fonctions de base comprend : une fonction de base de coefficient photoélectrique et une fonction de base de coefficient Compton.

7. Procédé selon la revendication 1, dans lequel le modèle de réseau de neurones pré-entraîné est un modèle obtenu en utilisant le groupe de seuils optimal local comme sortie d'un modèle de réseau de neurones et en utilisant les données de détection correspondant à chacun des rayons sélectionnés comme entrée du modèle de réseau de neurones,
dans lequel le groupe de seuils optimal local comprend des seuils pour l'imagerie K-edge du détecteur de comptage photonique déterminés par une méthode d'énumération lorsqu'une limite inférieure de bruit de la fonction de base du matériau d'agent de contraste est minimale, la limite inférieure de bruit de la fonction de base du matériau d'agent de contraste étant déterminée sur la base d'une théorie de limite inférieure Cramer-Rao.

8. Appareil (800, 900) d'optimisation de seuils basé sur l'imagerie K-edge, comprenant :

un module de détermination de valeur d'intégration linaire (810), un module de sélection (820), un module de modèle de réseau de neurones pré-entraîné (830) et un module d'intégration (840) ;
le module de détermination de valeur d'intégration linaire (810) étant configuré pour déterminer, en fonction des premières données de détection, des valeurs d'intégration linaire de coefficients de décomposition de fonctions de base individuelles dans une combinaison de fonctions de base de chaque rayon, dans lequel chaque rayon correspond à une combinaison de fonctions de base fixe ; et les premières données de détection sont obtenues en pré-balayant, par le détecteur de comptage photonique, un objet à détecter selon un groupe de seuils prédéfinis ;
le module de sélection (820) étant configuré pour sélectionner, en fonction des valeurs d'intégration linaire des coefficients de décomposition des fonctions de base individuelles dans la combinaison de fonctions de base de chaque rayon, des rayons dont une valeur d'intégration linaire de la fonction de base d'un matériau d'agent de contraste dans la combinaison de fonctions de base fixe est supérieure à un seuil d'intégration linaire prédéfini ;
le module de modèle de réseau de neurones pré-entraîné (830) étant configuré pour entrer des données de détection correspondant à chacun des rayons sélectionnés dans un modèle de réseau de neurones pré-entraîné, de sorte d'obtenir un groupe de seuils optimal local de chacun des rayons sélectionnés ; et
le module d'intégration (840) étant configuré pour déterminer, sur la base des groupes de seuils optimaux locaux, un groupe de seuils optimal global pour l'imagerie K-edge du détecteur de comptage photonique.

9. Appareil (800, 900) selon la revendication 8, dans lequel le groupe de seuils prédéfinis comprend des seuils fixes et des seuils à optimiser, les seuils à optimiser étant obtenus par une méthode d'équipartition énergétique.

10. Appareil (800, 900) selon la revendication 9, comprenant en outre :

un module de décomposition et/ou reconstruction (850),
le module de décomposition et/ou reconstruction (850) étant configuré pour décomposer et/ou reconstruire une image de l'objet à détecter sur la base des deuxièmes données de détection, dans lequel les deuxièmes données de détection sont obtenues en balayant, par le détecteur de comptage photonique, l'objet à détecter avec les seuils à optimiser remplacés par le groupe de seuil optimal global.

11. Appareil (800, 900) selon la revendication 8, dans lequel le module d'intégration (840) est configuré en outre pour : compter un nombre de rayons pour chacun des groupes de seuils optimaux locaux, et utiliser un groupe de seuils optimal local correspondant au plus grand nombre de rayons comme groupe de seuils optimal global.

12. Appareil (800, 900) selon la revendication 8, dans lequel le module de détermination de valeur d'intégration linaire (810) est configuré en outre pour :
calculer les premières données de détection par une méthode de décomposition de vraisemblance maximale, de sorte de déterminer les valeurs d'intégration linaire des coefficients de décomposition des fonctions de base individuelles dans la combinaison de fonctions de base de chaque rayon.

13. Appareil (800, 900) selon la revendication 8, dans lequel la combinaison de fonctions de base comprend : une fonction de base de coefficient photoélectrique et une fonction de base de coefficient Compton.

14. Appareil (800, 900) selon la revendication 8, dans lequel le modèle de réseau de neurones pré-entraîné est un modèle obtenu en utilisant le groupe de seuils optimal local comme sortie d'un modèle de réseau de neurones et en utilisant les données de détection correspondant à chacun des rayons sélectionnés comme entrée du modèle de réseau de neurones, dans lequel le groupe de seuils optimal local comprend des seuils pour l'imagerie K-edge du détecteur de comptage photonique déterminés par une méthode d'énumération lorsqu'une limite inférieure de bruit de la fonction de base du matériau d'agent de contraste est minimale, la limite inférieure de bruit de la fonction de base du matériau d'agent de contraste étant déterminée sur la base d'une théorie de limite inférieure Cramer-Rao.

15. Support de stockage de comptage comprenant des instructions qui, lorsqu'elles sont exécutées sur un ordinateur, amènent l'ordinateur à exécuter le procédé d'optimisation de seuils selon l'une quelconque des revendications 1 à 7.

Pre-scanning, by using a photon counting detector, an object to be detected according to a preset threshold group, to obtain first detection data ⟍╱ S110

Determining, according to the first detection data, line integral values of decomposition coefficients of individual basis functions in a basis function combination of each ray, wherein each ray has a fixed basis function combination ⟍╱ S120

Selecting, according to the line integral values of the decomposition coefficients of the individual basis functions in the basis function combination of the each ray, rays of which a line integral value of a contrast agent material basis function in the fixed basis function combination is greater than a preset line integration threshold ⟍╱ S130

Inputting detection data corresponding to each of the selected rays to a pre-trained neural network model, to obtain a local optimal threshold group of each of the selected rays ⟍╱ S140

Determining, based on local optimal threshold groups, a global optimal threshold group for the K-edge imaging of the photon counting detector ⟍╱ S150

**Fig. 1**

**Fig. 2**

Pre-scanning, by using a photon counting detector, an object to be detected according to a preset threshold group, to obtain first detection data ⟶ S110

Determining, according to the first detection data, line integral values of decomposition coefficients of individual basis functions in a basis function combination of each ray, wherein each ray has a fixed basis function combination ⟶ S120

Selecting, according to the line integral values of the decomposition coefficients of the individual basis functions in the basis function combination of the each ray, rays of which a line integral value of a contrast agent material basis function in the fixed basis function combination is greater than a preset line integration threshold ⟶ S130

Inputting detection data corresponding to each of the selected rays to a pre-trained neural network model, to obtain a local optimal threshold group of each of the selected rays ⟶ S140

Determining, based on local optimal threshold groups, a global optimal threshold group for the K-edge imaging of the photon counting detector ⟶ S150

Obtaining second detection data by using the photon counting detector to scan the object to be detected with the thresholds to be optimized replaced by the global optimal threshold group ⟶ S160

Decomposing and/or reconstructing an image of the object to be detected based on the second detection data ⟶ S170

**Fig. 3**

Fig. 4

Fig. 5

**Fig. 6**

Input
layer

$D_0(E')$

$D_1(E')$

$D_2(E')$

$D_3(E')$

Hidden
layer

1

Hidden
layer

2

Output
layer

Optimi
-zed
thresho
-lds

**Fig. 7**

**Fig. 8**

900

Line integral value determining
module 810

Selecting module 820

Pre-trained neural network
model module 830

Integrating module 840

Decomposing and/or
reconstructing module 850

**Fig. 9**

Computing device 1000

Input device 1001

Output device
1006

Input interface
1002

Central processor
1003

Memory 1004

Output interface
1005

Bus 1010

**Fig. 10**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2016054453 A1